# EUROPEAN PATENT APPLICATION

(11) **EP 3 836 153 A1**
(43) Date of publication of application: **16.06.2021**
(21) Application number: 19215213.0
(22) Date of filing: 11.12.2019
(51) Int. Cl.: G16H 20/30

(54) **SYSTEMS AND METHODS FOR AUDIBLE SLEEP THERAPY**

(71) Applicant: Koninklijke Philips N.V., 5656 AG Eindhoven (NL)
(72) Inventor: BRESCH, Erik, 5656 AE Eindhoven (NL); GROSSEKATHOEFER, Ulf, 5656 AE Eindhoven (NL)
(74) Representative: Philips Intellectual Property & Standards

(57) **Abstract**

The invention provides a system for providing an audio stimulus to a sleeping user. The system includes a user sensor adapted to acquire user sleep data from the sleeping user and a processor, which is adapted to determine a sleep stage of the sleeping user based on the user sleep data. The processor is further adapted to determine a predicted influence of an audio stimulus on the sleep stage of the sleeping user and generate a control signal based on the predicted influence of the audio stimulus. The system further comprises an audio output device, adapted to receive the control signal and generate an audio stimulus based on the control signal.

## Description

### FIELD OF THE INVENTION

The invention relates to the field of audible sleep therapy, and more specifically to the field of predictive adjustment of audible sleep therapy.

### BACKGROUND OF THE INVENTION

Sleep therapy with audio stimulation requires temporally predictive real-time sleep staging in order to make decisions regarding audio stimuli to provide to a sleeping user. The audio stimulus itself can alter the sleep stage of the user in a negative or positive manner.

Auditory sleep therapy aims to improve a person's sleep pattern, for example by increasing the time spent in deep sleep, by means of real-time auditory stimulation. The audio stimulus must be provided to the user at the right time, such as when the person is in deep sleep. Otherwise, the effect of auditory stimuli be destructive rather than beneficial by disturbing the sleep of the user.

Current implementations of stimulation logic trigger auditory stimuli based on hand-designed fixed rules given the current or expected future sleep stage. However, current audio stimulus based sleep therapy systems do not account for the potential effects of the audio stimulus on the user's current sleep stage.

There is therefore a need for a sleep therapy system with improved audio stimulus selection.

### SUMMARY OF THE INVENTION

The invention is defined by the claims.

According to examples in accordance with an aspect of the invention, there is provided a system for providing an audio stimulus to a sleeping user, the system comprising:
a user sensor adapted to acquire user sleep data from the sleeping user;
a processor adapted to:
   determine a sleep stage of the sleeping user based on the user sleep data;
   determine a predicted influence of an audio stimulus on the sleep stage of the sleeping user; and
   generate a control signal based on the predicted influence of the audio stimulus; and
an audio output device, adapted to receive the control signal and generate an audio stimulus based on the control signal.

The system provides a means of predicting the effect of an audio stimulus, for example as part of an auditory sleep therapy system, before providing the audio stimulus to the user.

In this way, it is possible to avoid negative effects of audio stimuli being provided to a user at a less optimal time or sleep state.

In an embodiment, determining the predicted influence of the audio stimulus on the sleep stage comprises:
simulating a first predicted user response to being provided the audio stimulus;
simulating a second predicted user response to not being provided the audio stimulus;
comparing the first predicted user response and the second predicted user response; and
determining the predicted influence of the audio stimulus based on the comparison.

In this way, the system may be adapted to decide whether it is preferable to provide the audio stimulus to the user or to not provide the audio stimulus to the user.

In an embodiment, the audio stimulus comprises a plurality of audio signals, and wherein determining the predicted influence of the audio stimulus on the sleep stage comprises:
for each of the plurality of audio signals, simulating a predicted user response to being provided the audio signal, thereby simulating a plurality of predicted user responses;
simulating an additional user response to not being provided the audio stimulus;
comparing each predicted user response of the plurality of predicted user responses to the other predicted user responses and the additional user response; and
determining the predicted influence of the audio stimulus based on the comparison.

In this way, the system may be adapted to compare a plurality of different types of audio stimuli in order to identify the most effective stimulus to be provided to the user.

In an embodiment, determining the predicted influence of the audio stimulus on the sleep stage is performed using a neural network.

In this way, the system may be trained to generate the audio stimulus based on an individual user, using a given set of user data.

In an embodiment, the system is further adapted to monitor a true user response to the generated audio stimulus, by way of the user sensor, and wherein determining the predicted influence of the audio stimulus on the sleep stage is further based on the true user response.

In this way, the system may be adapted to generate an audio stimulus based on measured effects of audio stimuli provided to the user.

In an embodiment, generating the control signal comprises:
determining an adjustment to a parameter of the audio stimulus based on the predicted influence of the audio stimulus; and
providing the adjustment as part of the control signal.

In an embodiment, the parameter of the audio stimulus comprises one or more of:
a volume;
a frequency;
a timbre; and
a duration.

In an embodiment, the user sensor comprises an EEG monitor and the user sleep data comprises EEG data.

According to examples in accordance with an aspect of the invention, there is provided a method for providing an audio stimulus to a sleeping user, the method comprising:
obtaining user sleep data relating to a sleeping user;
determining a sleep stage of the sleeping user based on the user sleep data;
determining a predicted influence of an audio stimulus on the sleep stage of the sleeping user; and
generating an audio stimulus based on the predicted influence of the audio stimulus.

In an embodiment, determining the predicted influence of the audio stimulus on the sleep stage comprises:
simulating a first predicted user response to being provided the audio stimulus;
simulating a second predicted user response to not being provided the audio stimulus;
comparing the first predicted user response and the second predicted user response; and
determining the predicted influence of the audio stimulus based on the comparison.

In an embodiment, the audio stimulus comprises a plurality of audio signals, and wherein determining the predicted influence of the audio stimulus on the sleep stage comprises:
for each of the plurality of audio signals, simulating a predicted user response to being provided the audio signal, thereby simulating a plurality of predicted user responses;
simulating an additional user response to not being provided the audio stimulus;
comparing each predicted user response of the plurality of predicted user responses to the other predicted user responses and the additional user response; and
determining the predicted influence of the audio stimulus based on the comparison.

In an embodiment, determining the predicted influence of the audio stimulus on the sleep stage is performed using a neural network.

In an embodiment, the method further comprises monitoring a true user response to the generated audio stimulus, and wherein determining the predicted influence of the audio stimulus on the sleep stage is further based on the true user response.

In an embodiment, generating the audio stimulus comprises:
determining an adjustment to a parameter of the audio stimulus based on the predicted influence of the audio stimulus; and
generating an adjusted audio stimulus based on the determined adjustment.

According to examples in accordance with an aspect of the invention, there is provided a computer program comprising computer program code means which is adapted, when said computer program is run on a computer, to implement the methods described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 a schematic representation of a system for providing an audio stimulus to a sleeping user;
Figure 2 shows a method for providing an audio stimulus to a sleeping user; and
Figures 3 shows a method for determining a predicted influence of an audio stimulus on a sleep state of a sleeping user.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for providing an audio stimulus to a sleeping user. The system includes a user sensor adapted to acquire user sleep data from the sleeping user and a processor, which is adapted to determine a sleep stage of the sleeping user based on the user sleep data. The processor is further adapted to determine a predicted influence of an audio stimulus on the sleep stage of the sleeping user and generate a control signal based on the predicted influence of the audio stimulus. The system further comprises an audio output device, adapted to receive the control signal and generate an audio stimulus based on the control signal.

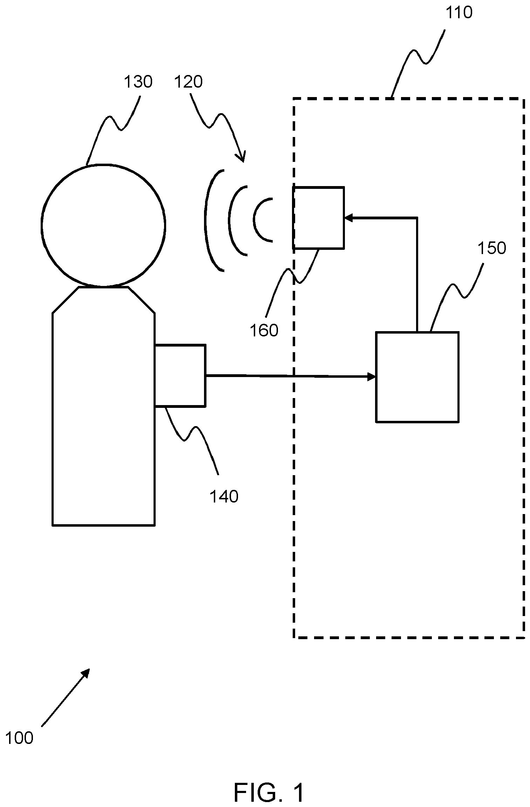

Figure 1 shows a schematic representation 100 of a system 110 for providing an audio stimulus 120 to a sleeping user 130.

The system 110 includes a user sensor 140 adapted to acquire user sleep data from the sleeping user 130. The user sensor 140 may be any sensor capable of obtaining data from the sleeping user. For example, the user sensor 140 may comprise an EEG monitor, meaning the user sleep data would comprise EEG data. Further, the user sensor may comprise one or more of: an ECG sensor; an EMG sensor; an accelerometer; a thermometer; a camera; a microphone; and the like.

Further, the system 110 comprises a processor 150 adapted to determine a sleep stage of the sleeping user based on the user sleep data. For example, user data may indicate that the sleeping user is in a given stage of deep sleep, such as stage N2 or stage N3, which may be identified from the user data by the processor.

The processor 150 is further adapted to determine a predicted influence of an audio stimulus 120 on the sleep stage of the sleeping user 130. In other words, the processor may determine whether or not the audio stimulus will result in a positive influence on the sleep stage of the sleeping user or a negative influence on the sleep stage of the sleeping user. For example, if the sleeping user were to be in an N2 stage of deep sleep, the processor may determine that the audio stimulus may bring the sleeping user out of deep sleep, which would be a negative influence should the system be trying to promote deep sleep. Alternatively, if the user were to be in an N3 stage of deep sleep, the processor may determine that the audio stimulus would result in the sleeping user maintaining the N3 stage of deep sleep for an extended period of time, which may be taken as a positive influence if the system is implemented to promote extended periods of deep sleep for the user.

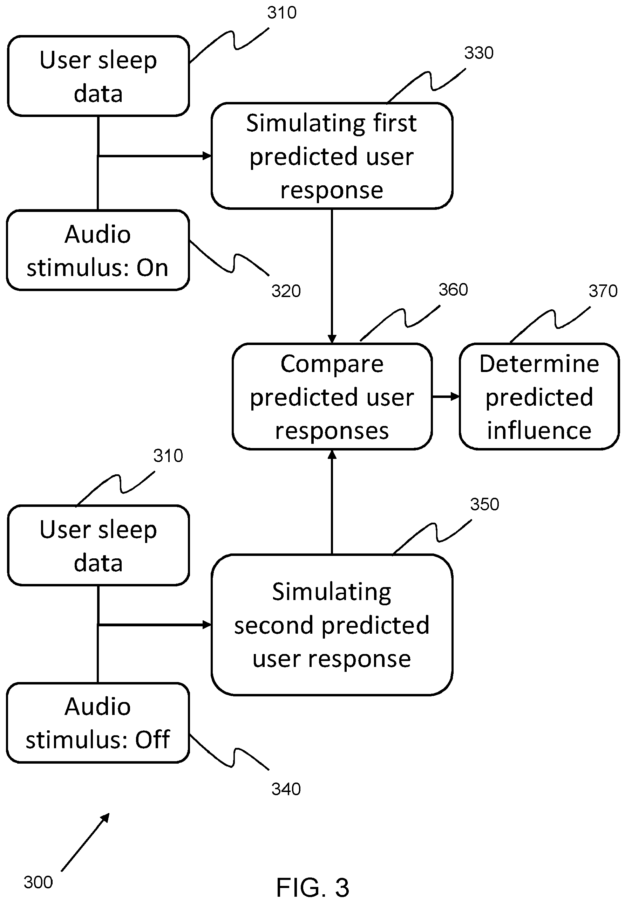

Various method for determining the predicted influence of the audio stimulus on the sleep stage of the sleeping user are discussed further below with reference to Figures 3a and 3b.

The system 110 further comprises an audio output device 160 and the processor 150 is adapted to generate a control signal to be provided to the audio output device based on the predicted influence of the audio stimulus, wherein the audio output device is adapted to generate an audio stimulus based on the control signal.

Generating the control signal may comprises determining an adjustment to a parameter of the audio stimulus based on the predicted influence of the audio stimulus and providing the adjustment as part of the control signal. The parameter for adjustment may comprise one or more of: a volume; a frequency; a timbre; and a duration.

For example, where the parameter in question is the volume parameter of the audio stimulus, in which case, the effect of different volumes can be tested in simulation to not wake up the user. In another example, where the parameter is the frequency of the audio stimulus the effect of the stimulation tone frequency on the sleep stage may be tested. Further, any combination of different parameters may be simulated to determine a predicted influence of the audio stimulus on the sleep stage of the user.

The system 100 may be further adapted to monitor the response of the sleeping user to the generated audio stimulus 120 by way of the user sensor 140. In other words, when the audio stimulus is provided to the sleeping user 130, the user data may be processed to determine the true influence of the audio stimulus on the sleep stage of the user. The true influence may then be compared to the predicted influence and the result of the comparison may be used in future predictions of the influence of the audio stimulus on the sleeping user.

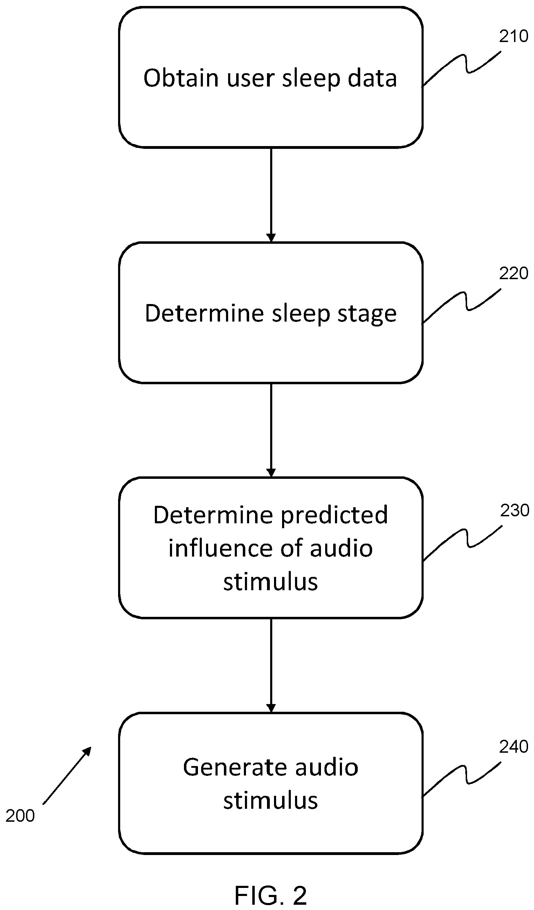

Figure 2 shows a method 200 for providing an audio stimulus to a sleeping user.

The method 200 begins in step 210 by obtaining user sleep data relating to a sleeping user. As described above, the user sleep data may include any data type relating to the sleeping user.

In step 220, a sleep stage of the sleeping user is determined based on the user sleep data. The step of determining the sleep stage of the sleeping user is described in detail further below with reference to Figures 3a and 3b.

In step 230, a predicted influence of an audio stimulus on the sleep stage of the sleeping user is determined.

In step 240, an audio stimulus is generated based on the predicted influence of the audio stimulus.

Figure 3 shows a method 300 for determining an influence of an audio stimulus of an audio stimulus on the sleep state of a user.

The user sleep data 310 and an activated audio stimulus 320 is provided to a simulation for simulating a first predicted user response 330 to being provided the audio stimulus.

Further, the user sleep data 310 and a deactivated audio stimulus 340 is provided to a simulation for simulating a second predicted user response 350 to not being provided the audio stimulus.

The first predicted user response and the second predicted user response are then compared in step 360 and the predicted influence of the audio stimulus determined in step 370 based on the result of the comparison.

The steps of determining the predicted influence of the audio stimulus on the sleeping user may be performed using a machine learning algorithm or neural network.

A machine-learning algorithm is any self-training algorithm that processes input data in order to produce or predict output data. Here, the input data comprises user sleep data and an audio stimulus, which comprises one or more parameters, and the output data comprises the predicted influence of the audio stimulus on the sleep state of the user.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example user sleep data and audio stimuli. The training output data entries correspond to predicted influences of the audio stimuli on a sleep state of a user. For example, the training input data may comprise one or more of: historic ECG data and stimulation data from other users; historic ECG data and stimulation data of the current user; and current ECG data and stimulation data from the current user.

In other words, there is provided a predictive real-time deep neural network sleep stager, which is able to forecast the effects of auditory stimulation on the sleep state of a sleeping user. In the description herein, such a network may be referred to as a stimulation effect predictor network (SEPN).

The SEPN features additional inputs, which a conventional deep neural network sleep stager does not account for, which are fed with hypothetical and/or actual stimulation decisions. The SEPN may be operated in two distinct modes, which are an exploratory/test prediction mode, as detailed above with respect to Figure 3, and a normal prediction mode.

In normal prediction mode, the current window of the ECG signal as well as the chosen audio stimulus settings are first applied to the network's inputs. Depending on the network's architecture (convolutional, dense, recurrent and the like), the internal mathematical computations are executed, which result in an update of the network outputs. The output traditionally consists of a continuous valued, "one-hot" - encoded vector with as many elements as there are sleep stages. The strength of each entry in the vector may correspond to a measure for the probability of the user being in the corresponding sleep stage.

These two operation modes may be used in an alternating fashion for each sleep epoch.

The exploratory mode is described above with reference to Figure 3. Here the SEPN is run twice (starting from the same internal state, which, in the case of an SEPN implementation with a recurrent network, summarizes the history of the sleep stages under consideration of the actually carried out stimulation actions): once with the audio stimulus input "off and once with the audio stimulus input "on". It should be noted that if more stimulation parameter choices exist, the described simulation may be run for each parameter choice to provide a full landscape of the predicted influence of the audio stimulus.

For example, in the case where the audio stimulus comprises a plurality of audio signals, determining the predicted influence of the audio stimulus on the sleep stage of the sleeping user may comprise, for each of the plurality of audio signals, simulating a predicted user response to being provided the audio signal, thereby simulating a plurality of predicted user responses. Each predicted user response of the plurality of predicted user responses may then be compared to the other predicted user responses and the additional user response to no audio stimulus. The predicted influence of the audio stimulus may then be determined based on the comparison.

The decision logic of the SEPN determines the predicted influence of the possible audio stimuli by considering predicted sleep stages, which may be in the form of hard or soft information from the user data, prior to actually executing, or not executing, the audio stimulus. For instance, the logic can chose the stimulation action that leads to the highest N3 soft-score.

When the audio stimulus has been selected for the sleeping user, the SEPN may be run again with the same set of user sleep data, such as the same EEG epoch, and with the chosen audio stimulus applied. This is the case for recurrent networks, which keep track of "state information" memory, which is an abstract summary of the user's past sleep states. Such a recurrent network's state memory needs to be updated with every time step, which happens when the network is run to predict the influence of an audio stimulus on the sleep state of the sleeping user.

Put another way, the SEPN may be used first to simulate the effect of all audio stimulus choices and subsequently, the most favorable audio stimulus is chosen. The audio stimulus is then finally executed with the selected stimulation choice, for example, to update the internal state if the SEPN network is a recurrent network.

An SEPN may be built from example data that includes all possible audio stimulus parameter choices and settings. This requires a sufficient amount of user sleep data, such as EEG recordings, with corresponding audio stimulus information and annotated sleep stages. The sleep stages may be obtained from sleep experts that examine the recorded user data in detail and determine the sleep stage of a window of user data. Sleep stages are traditionally assigned to short windows of user data of lengths 6 seconds or 30 seconds. However, the window may be of any size, or the user data may be continuously annotated.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (110) for providing an audio stimulus (120) to a sleeping user (130), the system comprising:
a user sensor (140) adapted to acquire user sleep data from the sleeping user;
a processor (150) adapted to:
determine a sleep stage of the sleeping user based on the user sleep data;
determine a predicted influence of an audio stimulus on the sleep stage of the sleeping user; and
generate a control signal based on the predicted influence of the audio stimulus; and
an audio output device (160), adapted to receive the control signal and generate an audio stimulus based on the control signal.

2. A system (110) as claimed in claim 1, wherein determining the predicted influence of the audio stimulus on the sleep stage comprises:
simulating a first predicted user response to being provided the audio stimulus;
simulating a second predicted user response to not being provided the audio stimulus;
comparing the first predicted user response and the second predicted user response; and
determining the predicted influence of the audio stimulus based on the comparison.

3. A system (110) as claimed in claim 1, wherein the audio stimulus comprises a plurality of audio signals, and wherein determining the predicted influence of the audio stimulus on the sleep stage comprises:
for each of the plurality of audio signals, simulating a predicted user response to being provided the audio signal, thereby simulating a plurality of predicted user responses;
simulating an additional user response to not being provided the audio stimulus;
comparing each predicted user response of the plurality of predicted user responses to the other predicted user responses and the additional user response; and
determining the predicted influence of the audio stimulus based on the comparison.

4. A system (110) as claimed in any of claims 1 to 3, wherein determining the predicted influence of the audio stimulus on the sleep stage is performed using a neural network.

5. A system (110) as claimed in any of claims 1 to 4, wherein the system is further adapted to monitor a true user response to the generated audio stimulus, by way of the user sensor, and wherein determining the predicted influence of the audio stimulus on the sleep stage is further based on the true user response.

6. A system (110) as claimed in any of claims 1 to 5, wherein generating the control signal comprises:
determining an adjustment to a parameter of the audio stimulus based on the predicted influence of the audio stimulus; and
providing the adjustment as part of the control signal.

7. A system (110) as claimed in claim 6, wherein the parameter of the audio stimulus comprises one or more of:
a volume;
a frequency;
a timbre; and
a duration.

8. A system (110) as claimed in any of claims 1 to 7, wherein the user sensor comprises an EEG monitor and the user sleep data comprises EEG data.

9. A method (200) for providing an audio stimulus to a sleeping user, the method comprising:
obtaining (210) user sleep data relating to a sleeping user;
determining (220) a sleep stage of the sleeping user based on the user sleep data;
determining (230) a predicted influence of an audio stimulus on the sleep stage of the sleeping user; and
generating (240) an audio stimulus based on the predicted influence of the audio stimulus.

10. A method (200) as claimed in claim 9, wherein determining the predicted influence of the audio stimulus on the sleep stage comprises:
simulating (330) a first predicted user response to being provided the audio stimulus;
simulating (340) a second predicted user response to not being provided the audio stimulus;
comparing (360) the first predicted user response and the second predicted user response; and
determining (370) the predicted influence of the audio stimulus based on the comparison.

11. A method (200) as claimed in claim 9, wherein the audio stimulus comprises a plurality of audio signals, and wherein determining the predicted influence of the audio stimulus on the sleep stage comprises:
for each of the plurality of audio signals, simulating a predicted user response to being provided the audio signal, thereby simulating a plurality of predicted user responses;
simulating an additional user response to not being provided the audio stimulus;
comparing each predicted user response of the plurality of predicted user responses to the other predicted user responses and the additional user response; and
determining the predicted influence of the audio stimulus based on the comparison.

12. A method (200) as claimed in any of claims 9 to 11, wherein determining the predicted influence of the audio stimulus on the sleep stage is performed using a neural network.

13. A method (200) as claimed in any of claims 9 to 12, wherein the method further comprises monitoring a true user response to the generated audio stimulus, and wherein determining the predicted influence of the audio stimulus on the sleep stage is further based on the true user response.

14. A method (200) as claimed in any of claims 9 to 13, wherein generating the audio stimulus comprises:
determining an adjustment to a parameter of the audio stimulus based on the predicted influence of the audio stimulus; and
generating an adjusted audio stimulus based on the determined adjustment.

15. A computer program comprising computer program code means which is adapted, when said computer program is run on a computer, to implement the method of any of claims 9 to 14.
